# EUROPEAN PATENT SPECIFICATION

(11) **EP 2 832 348 B1**
(45) Date of publication and mention of the grant of the patent: **04.03.2020**
(21) Application number: 12873112.2
(22) Date of filing: 12.12.2012
(51) Int. Cl.: A61K 9/08, A61K 9/19, A61K 47/22, A61K 47/10, A61P 1/16, A61P 29/00, A61P 5/30, A61P 31/04, A61P 31/12, A61P 35/00, A61P 39/06, A61P 9/10, A61P 9/00, A61P 9/06, A61P 7/02

(54) **METHOD FOR DISSOLVING FLAVONOID COMPOUND, CARBON GLYCOSIDE COMPOUND, OR STILBENE COMPOUND AND METHOD FOR PREPARING AN INJECTION OR A POWDER FOR INJECTION**
VERFAHREN ZUM AUFLÖSEN EINER FLAVONOIDVERBINDUNG, KOHLENSTOFFGLYCOSIDVERBINDUNG ODER STILBENVERBINDUNG SOWIE VERFAHREN ZUR HERSTELLUNG EINER INJEKTION ODER EINES PULVERS ZUR INJEKTION
PROCÉDÉ DE DISSOLUTION D'UN COMPOSÉ FLAVONOÏDE, D'UN COMPOSÉ DE GLYCOSIDE CARBONÉ OU D'UN COMPOSÉ DE STILBÈNE, ET PROCÉDÉ DE PRÉPARATION D'UNE INJECTION OU D'UNE POUDRE POUR INJECTION

(30) Priority: 31.03.2012 CN 201210092871
(43) Date of publication of application: 04.02.2015
(73) Proprietor: Kunming Pharmaceutical Corp., Kunming, Yunnan 650106 (CN)
(72) Inventor: CHEN, Yunjian, Kunming Yunnan 650106 (CN); YANG, Zhaoxiang, Kunming Yunnan 650106 (CN); ZHU, Ze, Kunming Yunnan 650106 (CN); FANG, Fang, Kunming Yunnan 650106 (CN); PU, Junxue, Kunming Yunnan 650106 (CN)
(74) Representative: Bösl, Raphael Konrad
(86) International application number: PCT/CN2012/086443
(87) International publication number: WO 2013/143322

(56) References cited:
- WO-A1-2007/124668
- WO-A1-2009/012551
- CN-A- 1 454 596
- CN-A- 101 002 816
- CN-A- 101 019 877
- CN-A- 101 062 044
- CN-A- 101 664 435
- CN-A- 102 091 114
- CN-A- 102 140 144
- CN-A- 102 140 144
- CN-A- 102 292 111
- US-A1- 2011 262 569

## Description

### FIELD OF THE INVENTION

The present invention relates to the field of pharmaceutical technology, and particularly relates to a method for dissolving a carbon glycoside compound, or a stilbene compound and a method for preparing an injection or a powder for injection.

### BACKGROUND OF THE INVENTION

Flavonoid compounds in natural products have structural and bioactive diversity, and have hepatoprotective, anti-inflammatory, estrogenic, antibacterial, antiviral, anti-tumor and anti-oxyradical effects. For example, quercetin, rutin, hyperin, breviscapine, puerarin, as well as total flavone of Pueraria and Ginkgo, etc. all exhibit a protective effect on ischemic brain injury; hyperin, silibinin, luteolin, and total flavone of Hippophae, etc,have a protective effect on myocardial ischemia injury; and puerarin, daidzein and total flavone of Ginkgo leaves, etc, can significantly reduce cerebrovascular resistance and myocardium oxygen consumption as well as the generation of lactic acid, and have a significant protective effect on myocardial anoxia injury. Puerarin, daidzein, licoflavone as well as total flavone of Hippophae and Sophora flavescens have an antiarrhythmic effect. Luteolin, quercetin, kaempferide, apigenin as well as 4,2',4',-trihydroxy-3'-isopentenyl chalcone and 7,4'-dihydroxy-3'-isopentenyl chalcone have an inhibitory effect on platelet aggregation and thrombosis caused by arachidonic acid, collagen, platelet-activating factor, thrombin and so on, but these flavonoid compounds generally have lower solubility in water.

There are also many active ingredients in the natural products which belong to carbon glycoside compounds, such as barbaloin, homoorientin, mangiferin, bergenin and aloesin or the like. Generally, these carbon glycoside compounds are very stable to acid and base, of which the glycosidic bonds are difficult to be cleaved completely with a chemical method; however, mangiferin and isomangiferin have a carbon glycosidic structure, with lower solubility in various solvents.

There are also an important class of stilbene compounds in the natural products, including diphenylethylene glycosides, diphenylethyl compounds, phenanthrenes and derivatives thereof. The diphenylethylene glycoside compounds mainly have antibacterial, antifungal, hypolipidemic, hepatoprotective, antioxidant, antithrombotic, coronary vessel-expanding, antihypertensive, anti-allergy, platelet aggregation-inhibiting, anti-tumor and anti-HIV and other effects, but also have effects of inhibiting activities of DNA polymerase, antagonism of estrogen and inhibiting the growth of human breast cancer. The activity of the diphenylethyl compounds mainly consists in antibacterial effect, antifungal effect, inhibition of HIV, inhibition of 5-lipoxygenase, inhibition of DNA polymerase β, regulation of troponin and cytotoxic activity. The main bioactivity of the phenanthrene compounds mainly consists in anti-platelet aggregation and anti-tumor effects, which have been reported to have inhibitory effects on liver cancer and Ehrlich ascites carcinoma, and show cytotoxic effects on human cancer cells A549, SK-OV-3 and HL-60. The diphenylethylene glycoside compounds are mainly monomer derivatives or polymers (such as polydatin, triptophenolide, rhapontin and deoxyrhapontin (methyl polydatin)) with resveratrol, piceatannol or hydrides thereof as basic units in structure, which are divided into resveratrol oligomer analogues, isorhapontigenin oligomer analogues, piceatannol oligomer analogues, resveratrol and oxyresveratrol oligomers, glycosides formed by resveratrol oligomers, of which the linking sugars are all glucoses with the number thereof varying from 1 to 3, and the forms of the glycoside are oxyglycoside and glycoside. The above stilbene compounds have lower solubility in water.

There are usually problems about low solubility, low bioavailability in most of the natural products, which to some extent limit the possibility of their development as new drugs. Therefore, one of the main challenges for getting new drugs to the market is to find a suitable method to improve the solubility of poorly soluble drugs and improve the bioavailability.

The drug solubility can be improved by simple means, such as salt-forming, using a mixed solvent, adding a solubilizer, etc., but also can be improved by more complicated preparation means and new techniques. Salt-forming is the most common process to increase the drug solubility. Most of the organic drugs are weak acids or weak bases, which are convered to salts and separated in an organic solution. It is generally unexpected in advance whether certain salt is stable in water which may be converted to an acid or a base or decomposed, how about its hygroscopicity, how about its solubility under different conditions, whether the toxicity is enhanced, and whether it is suitable for preparing a certain preparation and the like, which will be known whether the certain salt is suitable only after pharmacokinetics, pharmacodynamics, toxicity and other tests. When using a mixed solvent to prepare liquid preparations, injections and the like, the mixed solvent is often used to enhance the solubility of the poorly soluble drugs. The most common one is to add alcohols such as glycerol, ethanol, propylene glycol, polyethylene glycol and the like into water. However, because organic solvents would easily lead to pollution, affecting the health of operators, they are not widely used. As for adding a solubilizer to improve the drug solubility, when the addition of another small molecule substance to an aqueous solution of a drug enhances the drug solubility without reducing its physiological activity, this small molecule substance is referred to as a solubilizer. The mechanism of solubilization includes the formation of soluble complexes, complex salts, composites, etc. The common solubilizers include inorganic compounds such as potassium iodide and the like, organic acids and sodium salts thereof such as sodium benzoate, sodium salicylate and the like, as well as amines or amides such as ethylenediamine, urea, acetamide and nicotinamide. However, the dissolution speed of the foregoing solubilizers is not ideal, and the composition of the prepared water-soluble drug is unstable and it has low drug content. The new technologies for improving the solubility of poorly soluble drugs mainly include micellar solubilization, solid dispersion, inclusion compound and the like, which can not only increase the solubility of the drugs, but also may cover up the objectionable odor and taste of drugs, reduce the irritation and toxicity, provide sustained release of drugs, and improve the stability and bioavailability of drugs, etc. However, the new technologies for improving the solubility of poorly soluble drugs have complex operations and a relatively slow dissolution speed, and the redissolution speed of the prepared water-soluble drug is not very ideal, with the fastest dissolution speed above 10 s, limiting industrial production of the drug.

In WO 2007/124668, a pharmaceutical composition comprising a high concentration of polydatin is provided. The composition contains polydatin, meglumine and/or cyclodextrin. The composition is in the form of an aqueous solution or lyophilized product which is to be prepared before use. In CN 101 019 877, a mangiferin inclusion compound having greater solubility in water and a method for preparing the same are provided. The invention solves the technical problem of the low solubility of mangiferin in water and adopts the inclusion technique to increase the solubility of mangiferin in water.

Therefore, a solubilizer which is capable of increasing the solubility of poorly soluble drugs, is provided which has a fast redissolution speed, safety and is suitable for industrial production, and is of practical significance.

### SUMMARY OF THE INVENTION

In view of this, the present invention provides a method for dissolving a carbon glycoside compound or a stilbene compound, characterized in that the method comprises the following steps:
heating water for injection to 80-98 °C, followed by stirring under vacuum condition at 30-80 °C, and then adding the carbon glycoside compound or the stilbene compound along with a solubilizer under the protection of nitrogen gas or argon gas to obtain a solution of said compounds;
wherein the solubilizer is one or a mixture of two or more of gastrodin, water-soluble cyclodextrin and ethanol, and wherein the water-soluble cyclodextrin is one or a mixture of two or more of methyl-β-cyclodextrin, thioether-β-cyclodextrin and sulfobutyl ether-β-cyclodextrin; and
wherein the mass ratio of the carbon glycoside compound or the stilbene compound to the solubilizer is 1:1-20.

Disclosed is a method for dissolving a flavonoid compound, a carbon glycoside compound, or a stilbene compound and a method for preparing an injection or a powder for injection. The method includes: obtaining a reactant after mixing the flavonoid compound, the carbon glycoside compound, or the stilbene compound with a solubilizer; heating water for injection and stirring in vacuum, then adding the reactant under the protection of nitrogen gas or argon gas, to obtain a product.

The dissolution speed of the above methods is fast, and redissolution tests show that the prepared freeze-dried powder after freeze-drying the water soluble drug can be dissolved completely in only five seconds, has a significant increase in the redissolution speed as compared with a control group; also, the stability is good, which meets the requirement for clinical emergencies.

Disclosed is a method for dissolving a flavonoid compound, a carbon glycoside compound or a stilbene compound, including:
mixing the flavonoid compound, the carbon glycoside compound or the stilbene compound with a solubilizer to obtain a reactant; and
heating water for injection to 80-98 °C, followed by stirring under vacuum conditions at 30-80 °C, and then adding the reactant under the protection of nitrogen gas or argon gas to obtain a solution of the reactant.

Considering the sensitivity to heat or light of the flavonoid compound, the carbon glycoside compound or the stilbene compound, a method is provided by heating water for injection to 80-98 °C to remove dissolved oxygen and other gases in water, which reduces the surface tension of water, increases the contact area of water with the flavonoid compound, the carbon glycoside compound or the stilbene compound, thus increasing the dissolution speed. Meanwhile, pre-heating the water for injection can effectively avoid degradation of the flavonoid compound, the carbon glycoside compound or the stilbene compound due to exposure to the dissolved oxygen, to eliminate unstable factors of the flavonoid compound, the carbon glycoside compound or the stilbene compound. The vacuum conditions can achieve further deoxygenation, facilitating the dissolution of the flavonoid compound, the carbon glycoside compound or the stilbene compound in water. Nitrogen gas or argon gas provides an inert atmosphere, which further reduces the surface tension of the materials, increases the contact area of the flavonoid compound, the carbon glycoside compound or the stilbene compound with water, and increases the dissolution speed.

Stirring can accelerate the removal of the dissolved oxygen in water, thus improving the dissolution speed. However, due to the instability of the flavonoid compound, the carbon glycoside compound or the stilbene compound, stirring for too long time will easily lead to the degradation of the flavonoid compound, the carbon glycoside compound or the stilbene compound. Therefore, controlling the stirring time may increase the dissolution speed while keeping the flavonoid compound, the carbon glycoside compound or the stilbene compound stable. Preferably, the stirring time is 1-60 min.

Preferably, the solubilizer is one or a mixture of two or more of gastrodin, water-soluble cyclodextrin and ethanol.

Preferably, the water-soluble cyclodextrin includes one or a mixture of two or more of α-cyclodextrin derivatives, β-cyclodextrin derivatives, γ-cyclodextrin derivatives with various degrees of substitution.

Preferably, the water-soluble cyclodextrin includes one or a mixture of two or more of methyl-β-cyclodextrin, hydroxypropyl-β-cyclodextrin, thioether-β-cyclodextrin, sulfobutyl ether-β-cyclodextrin.

The flavonoid compound may be selected from the group consisting of flavone, isoflavone, flavonol, dihydroisoflavone, dihydroflavone, isoflavane, dihydroflavonol, rotenone, anthocyanin, pterocarpin, flavan-3-ol, aurone, flavan-3,4-diol, isoaurone, chalcone, homoflavone, dihydrochalcone, phenethylchromone, xanthone or homoisoflavone.

The flavone may be selected from the group consisting of baicalin, baicalein, wogonin or breviscapine; the isoflavone is selected from the group consisting of daidzein, daidzin or puerarin; the flavonol is selected from the group consisting of kaempferide or quercetin; the dihydroflavone is selected from the group consisting of dihydrobaicalein, taxinine, naringenin or hesperetin; the pterocarpin can be maackiain; and the homoisoflavone can be homoisoflavone.

Preferably, the carbon glycoside compound is selected from the group consisting of barbaloin, homoorientin, mangiferin, bergenin or aloesin.

Preferably, the stilbene compound is selected from the group consisting of resveratrol, piceatannol, polydatin, triptophenolide, rhapontin, deoxyrhapontin, as well as a polymer formed by one or more of resveratrol, piceatannol, polydatin, triptophenolide, rhapontin, deoxyrhapontin.

The stilbene compound refers to a general term of monomers and polymers having 1,2-diphenylethene and derivates thereof, including diphenylethylene glycosides, diphenylethyl compounds, phenanthrenes and derivatives thereof. The diphenylethylene glycoside compounds mainly have antibacterial, antifungal, hypolipidemic, hepatoprotective, antioxidant, antithrombotic, coronary vessel-expanding, antihypertensive, anti-allergy, platelet aggregation-inhibiting, anti-tumor and anti-HIV and other effects, but also have effects of inhibiting activities of DNA polymerase, antagonism of estrogen and inhibiting the growth of human breast cancer.

The diphenylethylene glycoside compounds are mainly monomer derivatives or polymers (such as polydatin, triptophenolide, rhapontin and deoxyrhapontin [methyl polydatin]) with resveratrol, piceatannol or hydrides thereof as basic units in structure, which are divided into resveratrol oligomer analogues, isorhapontigenin oligomer analogues, piceatannol oligomer analogues, resveratrol and oxyresveratrol oligomers, glycosides formed by resveratrol oligomers, of which the linking sugars are all glucoses with the number varying from 1 to 3, and the glycosides have the form of oxyglycoside and glycoside. Preferably, the stilbene compound is selected from the group consisting of resveratrol, piceatannol, polydatin, triptophenolide, rhapontin, dexoyrhapontin, as well as a polymer formed by one or more of resveratrol, piceatannol, polydatin, triptophenolide, rhapontin, dexoyrhapontin.

Preferably, the stilbene compound is triptophenolide, polydatin, resveratrol, piceatannol, rhapontin or dexoyrhapontin.

Triptophenolide is a compound of (E)-1-(3,5-dihydroxyphenyl)-2-(3-hydroxy-4-O-β-D-glucopyranosylphenyl)ethylene, or called 3,5,3',4'-tetrahydroxystilbene -3'-O-β-glucoside (also called 3,5,4'-trihydroxy-stilbene-3'-glucoside), and the chemical structure thereof is shown in formula I:

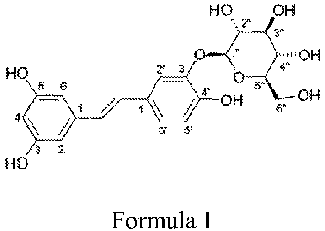

Disclosed is a mass ratio of the flavonoid compound, the carbon glycoside compound or the stilbene compound to the solubilizer of 1:0.1-80.

According to the invention, the mass ratio of the carbon glycoside compound or the stilbene compound to the solubilizer is 1:1-20.

Preferably, the mass ratio of the carbon glycoside compound or the stilbene compound to the solubilizer is 1:3-10.

Preferably, the mass-to-volume ratio of the solubilizer to the solution of the reactant is 2%-60% in g/mL.

Preferably, the mass-to-volume ratio of the solubilizer to the solution of the reactant is 3%-50% in g/mL.

Preferably, the mass-to-volume ratio of the solubilizer to the solution of the reactant is 3%-28% in g/mL.

In some embodiments of the present invention, the solubilizer is gastrodin and water-soluble cyclodextrin, and the mass-to-volume ratio of the solubilizer to the solution of the reactant is 11%-15% in g/mL.

In other embodiments of the present invention, the solubilizer is only gastrodin, and the mass-to-volume ratio of the solubilizer to the solution of the reactant is 8%-20% in g/mL.

In other embodiments of the present invention, the solubilizer is only water-soluble cyclodextrin, and the mass-to-volume ratio of the solubilizer to the solution of the reactant is 3%-8% in g/mL.

The present invention provides a method for preparing an injection of a carbon glycoside compound or a stilbene compound, characterized in that the method comprises the following steps:
heating water for injection to 80-98 °C, followed by stirring under vacuum condition at 30-80 °C, and then adding the carbon glycoside compound or the stilbene compound along with a solubilizer under the protection of nitrogen gas or argon gas to obtain a solution of said compounds; and adjusting pH value, filtering, filling and sterilizing to obtain the injection;
wherein the solubilizer is one or a mixture of two or more of gastrodin, water-soluble cyclodextrin and ethanol, and wherein the water-soluble cyclodextrin is one or a mixture of two or more of methyl-β-cyclodextrin, thioether-β-cyclodextrin and sulfobutyl ether-β-cyclodextrin; and
wherein the mass ratio of the carbon glycoside compound or the stilbene compound to the solubilizer is 1:1-20.

Disclosed is a method for preparing an injection of a flavonoid compound, a carbon glycoside compound or a stilbene compound, including:
mixing the flavonoid compound, the carbon glycoside compound or the stilbene compound with a solubilizer to obtain a reactant;
heating water for injection to 80-98 °C, followed by stirring under vacuum conditions at 30-80 °C, and then adding the reactant under the protection of nitrogen gas or argon gas, to obtain a solution of the reactant; and removing the vacuum, removing the protection of nitrogen gas or argon gas, filtering, filling and sterilizing, to obtain the injection.

Preferably, the stirring time is 1-60 min.

The present invention also provides a method for preparing a powder for injection of a carbon glycoside compound or a stilbene compound, characterized in that the method comprises the following steps:
heating water for injection to 80-98 °C, followed by stirring under vacuum conditions at 30-80 °C, and then adding the carbon glycoside compound or the stilbene compound along with a solubilizer under the protection of nitrogen gas or argon gas to obtain a solution of said compounds; and adjusting pH value, sterile-filtering, sterile-filling and freeze-drying to obtain the powder for injection,
wherein the solubilizer is one or a mixture of two or more of gastrodin, water-soluble cyclodextrin and ethanol, and wherein the water-soluble cyclodextrin is one or a mixture of two or more of methyl-β-cyclodextrin, thioether-β-cyclodextrin and sulfobutyl ether-β-cyclodextrin; and
wherein the mass ratio of the carbon glycoside compound or the stilbene compound to the solubilizer is 1:1-20.

Disclosed is a method for preparing a powder for injection of a flavonoid compound, a carbon glycoside compound or a stilbene compound, including:
mixing the flavonoid compound, the carbon glycoside compound or the stilbene compound with a solubilizer to obtain a reactant;
heating water for injection to 80-98 °C, followed by stirring under vacuum conditions at 30-80 °C, and then adding the reactant under the protection of nitrogen gas or argon gas, to obtain a solution of the reactant; and removing the vacuum, removing the protection of nitrogen gas or argon gas, filtering, filling, sterilizing and freeze-drying to obtain the powder for injection.

Preferably, the stirring time is 1-60 min.

Preferably, after the removing of the vacuum and the removing of the protection of nitrogen gas or argon gas, it further includes a step of adding an anti-oxidant, a complexing agent or an acidifying or alkalizing agent.

Preferably, the mass-to-volume ratio of the anti-oxidant to the solution of the reactant is 0.01-0.1% in g/mL.

Preferably, the anti-oxidant includes one or a mixture of two or more of sodium bisulfite, sodium sulfite, vitamin C, L-cysteine hydrochloride, glutathione or glutathione sodium.

Preferably, the mass-to-volume ratio of the complexing agent to the solution of the reactant is 0.003-1.0% in g/mL.

Preferably, the complexing agent includes calcium sodium edetate and/or disodium edetate.

Preferably, the solution of the reactant has a pH value ≥ 5.

Preferably, the solution of the reactant has a pH value of 6-8.

Preferably, the acidifying or alkalizing agent includes one or a mixture of two or more of hydrochloric acid, acetic acid, sodium hydroxide, sodium carbonate, sodium bicarbonate, citric acid, sodium citrate or citric acid.

Redissolution tests show that using gastrodin as the solubilizer respectively for a flavonoid compound, a carbon glycoside compound, and a stilbene compound provides fast dissolution speed, and the prepared freeze-dried powder after freeze-drying the water soluble drug can dissolve completely in only five seconds, and has a significant increase in the redissolution speed as compared with a control group. Stability tests show that a sample prepared from a traditional mixed solvent of PEG400 and mannitol has a degree of degradation above 8%, failing to meet the requirements of stability for pharmaceutical preparations; while in the present invention, the freeze-dried powder of water-soluble drugs prepared by using gastrodin and/or water-soluble cyclodextrin as the solubilizer for the flavonoid compound, the carbon glycoside compound, and the stilbene compound with a fast dissolution speed, has no change in properties and related substances, no significant change in pH and content as compared with that before standing still; and the stability is good, which meets the requirement for clinical emergencies.

### DETAILED EMBODIMENTS

The present invention discloses a method for dissolving a carbon glycoside compound or a stilbene compound and a method for preparing an injection or a powder for injection, and those skilled in the art can use the content herein for reference and achieve the present invention by appropriately improving the process parameters. It is to be particularly noted that all the similar replacements and modifications are obvious for those skilled in the art, which are deemed to be included in the present invention. The method and use of the present invention have been described by preferred examples, and related persons may obviously make modifications or appropriate changes or combinations to the method and use described herein without departing from the content, and scope of the present invention, to achieve and apply the technique of the present invention.

All of the drugs and formulations used in the method for dissolving a flavonoid compound, a carbon glycoside compound or a stilbene compound are commercially available.

The present invention will be further illustrated in conjunction with the following Examples:

### Example 1: Preparation of a powder for injection by dissolving a flavonoid compound (comparative example)

**Formula:**

| | |
|---|---|
| Breviscapine | 5 g |
| Gastrodin | 100 g |
| Sodium carbonate | 10 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing breviscapine along with gastrodin and sodium carbonate weighed according to the amounts in the formula by nitrogen gas into the preparation tank. Breviscapine dissolved in about 5 minutes, and then vacuum and nitrogen gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 2: Preparation of a powder for injection by dissolving a flavonoid compound (comparative example)

**Formula:**

| | |
|---|---|
| Breviscapine | 25 g |
| Gastrodin | 100 g |
| Hydroxypropyl-β-cyclodextrin | 50 g |
| Sodium carbonate | 1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing breviscapine along with gastrodin, hydroxypropyl-β-cyclodextrin and sodium carbonate weighed according to the amounts in the formula by argon gas into the preparation tank. Breviscapine dissolved in about 5 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 3: Preparation of a powder for injection by dissolving a flavonoid compound (comparative example)

**Formula:**

| | |
|---|---|
| Quercetin | 25 g |
| Gastrodin | 100 g |
| Methyl-β-cyclodextrin | 50 g |
| Sodium carbonate | 1 g |
| Water for injection | 1000 ml |

60% of the total amount of water for injection for preparation was added into a preparation tank, heated to 95 °C with slight boiling, and kept at 80 °C. The vacuum was initiated while stirring for 1 minute, followed by bringing quercetin along with gastrodin, methyl-β-cyclodextrin and sodium carbonate weighed according to the amounts in the formula by argon gas into the preparation tank. Quercetin dissolved in about 5 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 4: Preparation of a powder for injection by dissolving a flavonoid compound (comparative example)

**Formula:**

| | |
|---|---|
| Kaempferide | 25 g |
| Gastrodin | 100 g |
| Thioether-β-cyclodextrin | 50 g |
| Sodium carbonate | 1 g |
| Water for injection | 1000 ml |

60% of the total amount of water for injection for preparation was added into a preparation tank, heated to 98 °C with slight boiling, and kept at 80 °C. The vacuum was initiated while stirring for 5 minutes, followed by bringing kaempferide along with gastrodin, thioether-β-cyclodextrin and sodium carbonate weighed according to the amounts in the formula by argon gas into the preparation tank. Kaempferide dissolved in about 5 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 5: Preparation of a powder for injection by dissolving a flavonoid compound (comparative example)

**Formula:**

| | |
|---|---|
| Puerarin | 25 g |
| Gastrodin | 100 g |
| Sulfobutyl ether-β-cyclodextrin | 50 g |
| Sodium carbonate | 1 g |
| Water for injection | 1000 ml |

60% of the total amount of water for injection for preparation was added into a preparation tank, heated to 95 °C with slight boiling, and kept at 80 °C. The vacuum was initiated while stirring for 3 minutes, followed by bringing puerarin along with gastrodin, sulfobutyl ether-β-cyclodextrin and sodium carbonate weighed according to the amounts in the formula by argon gas into the preparation tank. Puerarin dissolved in about 5 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 6: Preparation of a powder for injection by dissolving a flavonoid compound (comparative example)

**Formula:**

| | |
|---|---|
| Daidzin | 10 g |
| Hydroxypropyl-β-cyclodextrin | 50 g |
| Sodium bicarbonate (or sodium carbonate) | 1 g |
| Water for injection | 1000 ml |

60% of the total amount of water for injection for preparation was added into a preparation tank, heated to 95 °C with slight boiling, and kept at 80 °C. The vacuum was initiated while stirring for 8 minutes, followed by bringing daidzin along with hydroxypropyl-β-cyclodextrin and sodium bicarbonate weighed according to the amounts in the formula by argon gas into the preparation tank. Puerarin dissolved in about 5 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 7: Preparation of a powder for injection by dissolving a flavonoid compound (comparative example)

**Formula:**

| | |
|---|---|
| Daidzein | 10 g |
| Methyl-β-cyclodextrin | 50 g |
| Sodium bicarbonate (or sodium carbonate) | 1 g |
| Water for injection | 1000 ml |

60% of the total amount of water for injection for preparation was added into a preparation tank, heated to 80 °C with slight boiling, and kept at 80 °C. The vacuum was initiated while stirring for 60 minutes, followed by bringing daidzein along with methyl-β-cyclodextrin and sodium bicarbonate weighed according to the amounts in the formula by argon gas into the preparation tank. Daidzein dissolved in about 5 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 8: Preparation of a powder for injection by dissolving a flavonoid compound (comparative example)

**Formula:**

| | |
|---|---|
| Wogonin | 10 g |
| Thioether-β-cyclodextrin | 50 g |
| Sodium bicarbonate (or sodium carbonate) | 1 g |
| Water for injection | 1000 ml |

60% of the total amount of water for injection for preparation was added into a preparation tank, heated to 85 °C with slight boiling, and kept at 80 °C. The vacuum was initiated while stirring for 30 minutes, followed by bringing wogonin along with thioether-β-cyclodextrin and sodium bicarbonate weighed according to the amounts in the formula by argon gas into the preparation tank. Breviscapine dissolved in about 5 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 9: Preparation of a powder for injection by dissolving a flavonoid compound (comparative example)

**Formula:**

| | |
|---|---|
| Baicalein | 10 g |
| Sulfobutyl ether-β-cyclodextrin | 50 g |
| Sodium bicarbonate (or sodium carbonate) | 1 g |
| Water for injection | 1000 ml |

60% of the total amount of water for injection for preparation was added into a preparation tank, heated to 90 °C with slight boiling, and kept at 80 °C. The vacuum was initiated while stirring for 20 minutes, followed by bringing baicalein along with sulfobutyl ether-β-cyclodextrin and sodium bicarbonate weighed according to the amounts in the formula by argon gas into the preparation tank. Baicalein dissolved in about 5 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 10: Preparation of an injection by dissolving a flavonoid compound (comparative example)

**Formula:**

| | |
|---|---|
| Baicalin | 5 g |
| Gastrodin | 100 g |
| Sodium bisulfite | 0.2 g |
| Disodium edetate | 0.1 g |
| Sodium carbonate | 5 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing baicalin along with gastrodin, disodium edetate and sodium carbonate weighed according to the amounts in the formula by nitrogen gas into the preparation tank. Baicalin dissolved in about 5 minutes, then vacuum and nitrogen gas were removed, and sodium bisulfite was added in a conventional way, followed by adjusting pH and content and adding water to the total amount, thus completing the preparation process. Next, it was filtered, filled and sterilized by a conventional process to obtain the injection.

### Example 11: Preparation of an injection by dissolving a flavonoid compound (comparative example)

**Formula:**

| | |
|---|---|
| Homoisoflavonoid | 5 g |
| Gastrodin | 100 g |
| Sodium sulfite | 0.2 g |
| Disodium edetate | 0.1 g |
| Sodium carbonate | 3 g |
| Water for injection | 1000 ml |

60% of the total amount of water for injection for preparation was added into a preparation tank, heated to 85 °C with slight boiling, and kept at 80 °C. The vacuum was initiated while stirring for 40 minutes, followed by bringing homoisoflavone along with gastrodin, disodium edetate and sodium carbonate weighed according to the amounts in the formula by nitrogen gas into the preparation tank. Homoisoflavonoid dissolved in about 5 minutes, then vacuum and nitrogen gas were removed, and sodium sulfite was added in a conventional way, followed by adjusting pH and content and adding water to the total amount, thus completing the preparation process. Next, it was filtered, filled and sterilized by a conventional process to obtain the injection.

### Example 12: Preparation of an injection by dissolving a flavonoid compound (comparative example)

**Formula:**

| | |
|---|---|
| Maackiain | 5 g |
| Gastrodin | 100 g |
| Vitamin C | 0.2 g |
| Disodium edetate | 0.1 g |
| Sodium carbonate | 3 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing maackiain along with gastrodin, disodium edetate and sodium carbonate weighed according to the amounts in the formula by nitrogen gas into the preparation tank. Maackiain dissolved in about 5 minutes, then vacuum and nitrogen gas were removed, and vitamin C was added in a conventional way, followed by adjusting pH and content and adding water to the total amount, thus completing the preparation process. Next, it was filtered, filled and sterilized by a conventional process to obtain the injection.

### Example 13: Preparation of an injection by dissolving a flavonoid compound (comparative example)

**Formula:**

| | |
|---|---|
| Dihydrobaicalein | 5 g |
| Gastrodin | 100 g |
| L-cysteine hydrochloride | 0.2 g |
| Disodium edetate | 0.1 g |
| Sodium carbonate | 3 g |
| Water for injection | 1000 ml |

60% of the total amount of water for injection for preparation was added into a preparation tank, heated to 90 °C with slight boiling, and kept at 80 °C. The vacuum was initiated while stirring for 25 minutes, followed by bringing dihydrobaicalein along with gastrodin, disodium edetate and sodium carbonate weighed according to the amounts in the formula by nitrogen gas into the preparation tank. Dihydrobaicalein dissolved in about 5 minutes, then vacuum and nitrogen gas were removed, and L-cysteine hydrochloride was added in a conventional way, followed by adjusting pH and content and adding water to the total amount, thus completing the preparation process. Next, it was filtered, filled and sterilized by a conventional process to obtain the injection.

### Example 14: Preparation of an injection by dissolving a flavonoid compound (comparative example)

**Formula:**

| | |
|---|---|
| Naringenin | 5 g |
| Gastrodin | 100 g |
| Glutathione | 0.2 g |
| Disodium edetate | 0.1 g |
| Sodium carbonate | 2 g |
| Water for injection | 1000 ml |

60% of the total amount of water for injection for preparation was added into a preparation tank, heated to 98 °C with slight boiling, and kept at 80 °C. The vacuum was initiated while stirring for 10 minutes, followed by bringing naringenin along with gastrodin, disodium edetate and sodium carbonate weighed according to the amounts in the formula by nitrogen gas into the preparation tank. Naringenin dissolved in about 5 minutes, then vacuum and nitrogen gas were removed, and glutathione was added in a conventional way, followed by adjusting pH and content and adding water to the total amount, thus completing the preparation process. Next, it was filtered, filled and sterilized by a conventional process to obtain the injection.

### Example 15: Preparation of an injection by dissolving a flavonoid compound (comparative example)

**Formula:**

| | |
|---|---|
| Hesperetin | 5 g |
| Gastrodin | 100 g |
| Glutathione sodium | 0.2 g |
| Disodium edetate | 0.1 g |
| Sodium carbonate | 2 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing hesperetin along with gastrodin, disodium edetate and sodium carbonate weighed according to the amounts in the formula by nitrogen gas into the preparation tank. Hesperetin dissolved in about 5 minutes, then vacuum and nitrogen gas were removed, and glutathione sodium was added in a conventional way, followed by adjusting pH and content and adding water to the total amount, thus completing the preparation process. Next, it was filtered, filled and sterilized by a conventional process to obtain the injection.

### Example 16: Preparation of an injection by dissolving a flavonoid compound (comparative example)

**Formula:**

| | |
|---|---|
| Taxinine | 12 g |
| Sulfobutyl ether-β-cyclodextrin | 40 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing taxinine along with sulfobutyl ether-β-cyclodextrin weighed according to the amounts in the formula by nitrogen gas into the preparation tank. Taxinine dissolved in about 5 minutes, then vacuum and nitrogen gas were removed, and sodium hydroxide or hydrochloric acid was added to adjust the pH of the solution of 6.0-6.5 in a conventional way, followed by adjusting content and adding water to the total amount, thus completing the preparation process. Next, it was filtered, filled and sterilized by a conventional process to obtain the injection.

### Example 17: Preparation of a powder for injection by dissolving a carbon glycoside compound

**Formula:**

| | |
|---|---|
| Mangiferin | 10 g |
| Gastrodin | 100 g |
| Sodium bicarbonate | 2 g |
| Water for injection | 1000 ml |

40% of the total amount of water for injection for preparation was added into a preparation tank, heated to 95 °C with slight boiling, and kept at 80 °C. The vacuum was initiated while stirring for 10 minutes, followed by bringing mangiferin along with gastrodin and sodium bicarbonate weighed according to the amounts in the formula by nitrogen gas into the preparation tank. Mangiferin dissolved in about 10 minutes, and then vacuum and nitrogen gas were removed, followed by adjusting pH and content by adding sodium hydroxide or hydrochloric acid in a conventional way, and adding water to the total amount, thus completing the preparation process. Next, it was sterile-filtered, sterile-filled and freeze-dried by a conventional process to obtain the freeze-dried powder for injection.

### Example 18: Preparation of a powder for injection by dissolving a carbon glycoside compound

**Formula:**

| | |
|---|---|
| Mangiferin | 25 g |
| Gastrodin | 100 g |
| Hydropropyl-β-cyclodextrin | 50 g |
| Sodium bicarbonate | 1 g |
| Water for injection | 1000 ml |

40% of the total amount of water for injection for preparation was added into a preparation tank, heated to 95 °C with slight boiling, and kept at 80 °C. The vacuum was initiated while stirring for 10 minutes, followed by bringing mangiferin along with gastrodin, hydropropyl-β-cyclodextrin and sodium bicarbonate weighed according to the amounts in the formula by argon gas into the preparation tank. Mangiferin dissolved in about 10 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 19: Preparation of a powder for injection by dissolving a carbon glycoside compound

**Formula:**

| | |
|---|---|
| Barbaloin | 25 g |
| Gastrodin | 100 g |
| Methyl-β-cyclodextrin | 50 g |
| Sodium bicarbonate | 1 g |
| Water for injection | 1000 ml |

40% of the total amount of water for injection for preparation was added into a preparation tank, heated to 95 °C with slight boiling, and kept at 80 °C. The vacuum was initiated while stirring for 10 minutes, followed by bringing barbaloin along with gastrodin, methyl-β-cyclodextrin and sodium bicarbonate weighed according to the amounts in the formula by argon gas into the preparation tank. Barbaloin dissolved in about 10 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 20: Preparation of a powder for injection by dissolving a carbon glycoside compound

**Formula:**

| | |
|---|---|
| Homoorientin | 25 g |
| Gastrodin | 100 g |
| Thioether-β-cyclodextrin | 50 g |
| Sodium bicarbonate | 1 g |
| Water for injection | 1000 ml |

40% of the total amount of water for injection for preparation was added into a preparation tank, heated to 80 °C with slight boiling, and kept at 80 °C. The vacuum was initiated while stirring for 60 minutes, followed by bringing homoorientin along with gastrodin, thioether-β-cyclodextrin and sodium bicarbonate weighed according to the amounts in the formula by argon gas into the preparation tank. Homoorientin dissolved in about 10 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 21: Preparation of a powder for injection by dissolving a carbon glycoside compound

**Formula:**

| | |
|---|---|
| Bergenin | 25 g |
| Gastrodin | 100 g |
| Sulfobutyl ether-β-cyclodextrin | 50 g |
| Sodium bicarbonate | 1 g |
| Water for injection | 1000 ml |

40% of the total amount of water for injection for preparation was added into a preparation tank, heated to 85 °C with slight boiling, and kept at 80 °C. The vacuum was initiated while stirring for 50 minutes, followed by bringing bergenin along with gastrodin, sulfobutyl ether-β-cyclodextrin and sodium bicarbonate weighed according to the amounts in the formula by argon gas into the preparation tank. Bergenin dissolved in about 10 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 22: Preparation of a powder for injection by dissolving a carbon glycoside compound

**Formula:**

| | |
|---|---|
| Aloesin | 10 g |
| Hydroxypropyl-β-cyclodextrin | 50 g |
| Sodium bicarbonate (or sodium carbonate) | 1 g |
| Water for injection | 1000 ml |

40% of the total amount of water for injection for preparation was added into a preparation tank, heated to 88 °C with slight boiling, and kept at 80 °C. The vacuum was initiated while stirring for 35 minutes, followed by bringing aloesin along with hydroxypropyl-β-cyclodextrin and sodium bicarbonate weighed according to the amounts in the formula by argon gas into the preparation tank. Aloesin dissolved in about 5 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 23: Preparation of a powder for injection by dissolving a carbon glycoside compound

**Formula:**

| | |
|---|---|
| Barbaloin | 10 g |
| Methyl-β-cyclodextrin | 50 g |
| Sodium bicarbonate (or sodium carbonate) | 1 g |
| Water for injection | 1000 ml |

40% of the total amount of water for injection for preparation was added into a preparation tank, heated to 98 °C with slight boiling, and kept at 80 °C. The vacuum was initiated while stirring for 10 minutes, followed by bringing barbaloin along with methyl-β-cyclodextrin and sodium bicarbonate weighed according to the amounts in the formula by argon gas into the preparation tank. Barbaloin dissolved in about 5 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 24: Preparation of a powder for injection by dissolving a carbon glycoside compound

**Formula:**

| | |
|---|---|
| Homoorientin | 10 g |
| Thioether-β-cyclodextrin | 50 g |
| Sodium bicarbonate (or sodium carbonate) | 1 g |
| Water for injection | 1000 ml |

40% of the total amount of water for injection for preparation was added into a preparation tank, heated to 95 °C with slight boiling, and kept at 80 °C. The vacuum was initiated while stirring for 10 minutes, followed by bringing homoorientin along with thioether-β-cyclodextrin and sodium bicarbonate weighed according to the amounts in the formula by argon gas into the preparation tank. Homoorientin dissolved in about 5 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 25: Preparation of a powder for injection by dissolving a carbon glycoside compound

**Formula:**

| | |
|---|---|
| Bergenin | 10 g |
| Sulfobutyl ether-β-cyclodextrin | 50 g |
| Sodium bicarbonate (or sodium carbonate) | 1 g |
| Water for injection | 1000 ml |

40% of the total amount of water for injection for preparation was added into a preparation tank, heated to 95 °C with slight boiling, and kept at 80 °C. The vacuum was initiated while stirring for 10 minutes, followed by bringing bergenin along with sulfobutyl ether-β-cyclodextrin and sodium bicarbonate weighed according to the amounts in the formula by argon gas into the preparation tank. Bergenin dissolved in about 5 minutes, and then vacuum and argon gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 26: Preparation of an injection by dissolving a carbon glycoside compound

**Formula:**

| | |
|---|---|
| Aloesin | 5 g |
| Gastrodin | 100 g |
| Sodium bisulfite | 0.2 g |
| Disodium edetate | 0.1 g |
| Water for injection | 1000 ml |

40% of the total amount of water for injection for preparation was added into a preparation tank, heated to 95 °C with slight boiling, and kept at 80 °C. The vacuum was initiated while stirring for 10 minutes, followed by bringing aloesin along with gastrodin, disodium edetate and sodium carbonate weighed according to the amounts in the formula by nitrogen gas into the preparation tank. Aloesin dissolved in about 10 minutes, then vacuum and nitrogen gas were removed, and sodium bisulfite was added in a conventional way, followed by adjusting pH and content and adding water to the total amount, thus completing the preparation process. Next, it was filtered, filled and sterilized by a conventional process to obtain the injection.

### Example 27: Preparation of an injection by dissolving a carbon glycoside compound

**Formula:**

| | |
|---|---|
| Mangiferin | 5 g |
| Gastrodin | 100 g |
| Sodium sulfite | 0.2 g |
| Disodium edetate | 0.1 g |
| Water for injection | 1000 ml |

40% of the total amount of water for injection for preparation was added into a preparation tank, heated to 95 °C with slight boiling, and kept at 80 °C. The vacuum was initiated while stirring for 10 minutes, followed by bringing mangiferin along with gastrodin, disodium edetate and sodium carbonate weighed according to the amounts in the formula by nitrogen gas into the preparation tank. Mangiferin dissolved in about 10 minutes, then vacuum and nitrogen gas were removed, and sodium carbonate was added in a conventional way, followed by adjusting pH and content and adding water to the total amount, thus completing the preparation process. Next, it was filtered, filled and sterilized by a conventional process to obtain the injection.

### Example 28: Preparation of an injection by dissolving a carbon glycoside compound

**Formula:**

| | |
|---|---|
| Bergenin | 5 g |
| Gastrodin | 100 g |
| Vitamin C | 0.2 g |
| Disodium edetate | 0.1 g |
| Water for injection | 1000 ml |

40% of the total amount of water for injection for preparation was added into a preparation tank, heated to 95 °C with slight boiling, and kept at 80 °C. The vacuum was initiated while stirring for 10 minutes, followed by bringing bergenin along with gastrodin, disodium edetate and sodium carbonate weighed according to the amounts in the formula by nitrogen gas into the preparation tank. Bergenin dissolved in about 10 minutes, then vacuum and nitrogen gas were removed, and vitamin C was added in a conventional way, sodium carbonate was added in a conventional way, followed by adjusting pH and content and adding water to the total amount, thus completing the preparation process. Next, it was filtered, filled and sterilized by a conventional process to obtain the injection.

### Example 29: Preparation of an injection by dissolving a carbon glycoside compound

**Formula:**

| | |
|---|---|
| Mangiferin | 5 g |
| Gastrodin | 100 g |
| L-cysteine hydrochloride | 0.2 g |
| Disodium edetate | 0.1 g |
| Water for injection | 1000 ml |

40% of the total amount of water for injection for preparation was added into a preparation tank, heated to 95 °C with slight boiling, and kept at 80 °C. The vacuum was initiated while stirring for 10 minutes, followed by bringing mangiferin along with gastrodin, disodium edetate and sodium carbonate weighed according to the amounts in the formula by nitrogen gas into the preparation tank. Mangiferin dissolved in about 10 minutes, then vacuum and nitrogen gas were removed, L-cysteine hydrochloride was added in a conventional way, and sodium carbonate was added in a conventional way, followed by adjusting pH and content and adding water to the total amount, thus completing the preparation process. Next, it was filtered, filled and sterilized by a conventional process to obtain the injection.

### Example 30: Preparation of an injection by dissolving a carbon glycoside compound

**Formula:**

| | |
|---|---|
| Barbaloin | 5 g |
| Gastrodin | 100 g |
| Glutathione | 0.2 g |
| Disodium edetate | 0.1 g |
| Water for injection | 1000 ml |

40% of the total amount of water for injection for preparation was added into a preparation tank, heated to 95 °C with slight boiling, and kept at 80 °C. The vacuum was initiated while stirring for 10 minutes, followed by bringing barbaloin along with gastrodin, disodium edetate and sodium carbonate weighed according to the amounts in the formula by nitrogen gas into the preparation tank. Barbaloin dissolved in about 10 minutes, then vacuum and nitrogen gas were removed, glutathione was added in a conventional way and sodium carbonate was added, followed by adjusting pH and content and adding water to the total amount, thus completing the preparation process. Next, it was filtered, filled and sterilized by a conventional process to obtain the injection.

### Example 31: Preparation of an injection by dissolving a carbon glycoside compound

**Formula:**

| | |
|---|---|
| Mangiferin | 5 g |
| Gastrodin | 100 g |
| Glutathione sodium | 0.2 g |
| Disodium edetate | 0.1 g |
| Water for injection | 1000 ml |

40% of the total amount of water for injection for preparation was added into a preparation tank, heated to 95 °C with slight boiling, and kept at 80 °C. The vacuum was initiated while stirring for 10 minutes, followed by bringing mangiferin along with gastrodin, disodium edetate and sodium carbonate weighed according to the amounts in the formula by nitrogen gas into the preparation tank. Mangiferin dissolved in about 10 minutes, then vacuum and nitrogen gas were removed, glutathione sodium was added in a conventional way and sodium carbonate was added, followed by adjusting pH and content and adding water to the total amount, thus completing the preparation process. Next, it was filtered, filled and sterilized by a conventional process to obtain the injection.

### Example 32: Preparation of a powder for injection by dissolving a carbon glycoside compound

**Formula:**

| | |
|---|---|
| Mangiferin | 2.0 g |
| Hydroxypropyl-β-cyclodextrin | 200 g |
| Water for injection | 1000 ml |

40% of the total amount of water for injection for preparation was added into a preparation tank, heated to 95 °C with slight boiling, and kept at 80 °C. The vacuum was initiated while stirring for 10 minutes, followed by bringing mangiferin along with hydroxypropyl-β-cyclodextrin weighed according to the amounts in the formula by argon gas into the preparation tank. Mangiferin dissolved in about 5 minutes, and then vacuum and nitrogen gas were removed, followed by adjusting pH and content and adding water to the total amount, thus completing the preparation process. Next, it was sterile-filtered, sterile-filled and freeze-dried by a conventional process to obtain the freeze-dried powder for injection.

### Example 33: Preparation of a powder for injection by dissolving a carbon glycoside compound

**Formula:**

| | |
|---|---|
| Mangiferin | 2.0 g |
| Ethanol | 100 ml |
| Water for injection | 1000 ml |

40% of the total amount of water for injection for preparation was added into a preparation tank, heated to 95 °C with slight boiling, and kept at 40 °C. The vacuum was initiated while stirring for 10 minutes, followed by bringing mangiferin along with ethanol weighed according to the amounts in the formula by argon gas into the preparation tank. Mangiferin dissolved in about 1 minute, and then vacuum and nitrogen gas were removed, followed by adjusting pH and content and adding water to the total amount, thus completing the preparation process. Next, it was filtered, filled and sterilized by a conventional process to obtain the injection.

### Example 34: Preparation of powder for injection by dissolving a carbon glycoside compound

**Formula:**

| | |
|---|---|
| Mangiferin | 2.0 g |
| Ethanol | 100 ml |
| Water for injection | 1000 ml |

40% of the total amount of water for injection for preparation and a prescribed amount in the formula of ethanol were added into a preparation tank, heated to 80 °C, and mangiferin in an amount of the formula was added to the solvent under stirring. Mangiferin dissolved in about 30 minutes, followed by adjusting pH and content and adding water to the total amount, thus completing the preparation process. Next, it was filtered, filled and sterilized by a conventional process to obtain the injection.

### Example 35: Preparation of a powder for injection by dissolving a stilbene compound

**Formula:**

| | |
|---|---|
| Triptophenolide | 5 g |
| Gastrodin | 80 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 36: Preparation of a powder for injection by dissolving a stilbene compound

**Formula:**

| | |
|---|---|
| Triptophenolide | 20 g |
| Gastrodin | 200 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 37: Preparation of a powder for injection by dissolving a stilbene compound

**Formula:**

| | |
|---|---|
| Triptophenolide | 10 g |
| Gastrodin | 80 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 38: Preparation of a powder for injection by dissolving a stilbene compound

**Formula:**

| | |
|---|---|
| Triptophenolide | 25 g |
| Gastrodin | 80 g |
| Hydroxypropyl-β-cyclodextrin | 30 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin and hydroxypropyl-β-cyclodextrin weighed according to the amounts in the formula by nitrogen gas into the preparation tank. Triptophenolide dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 39: Preparation of a powder for injection by dissolving a stilbene compound

**Formula:**

| | |
|---|---|
| Resveratrol | 25 g |
| Gastrodin | 80 g |
| Methyl-β-cyclodextrin | 30 g |
| Water for injection | 1000 ml |

60% of the total amount of water for injection for preparation was added into a preparation tank, heated to 80 °C with slight boiling, and kept at 80 °C. The vacuum was initiated while stirring for 60 minutes, followed by bringing resveratrol along with gastrodin and methyl-β-cyclodextrin weighed according to the amounts in the formula by nitrogen gas into the preparation tank. Resveratrol dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 40: Preparation of a powder for injection by dissolving a stilbene compound

**Formula:**

| | |
|---|---|
| Piceatannol | 25 g |
| Gastrodin | 80 g |
| Thioether-β-cyclodextrin | 30 g |
| Water for injection | 1000 ml |

60% of the total amount of water for injection for preparation was added into a preparation tank, heated to 90 °C with slight boiling, and kept at 80 °C. The vacuum was initiated while stirring for 20 minutes, followed by bringing piceatannol along with gastrodin and thioether-β-cyclodextrin weighed according to the amounts in the formula by nitrogen gas into the preparation tank. Piceatannol dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 41: Preparation of a powder for injection by dissolving a stilbene compound

**Formula:**

| | |
|---|---|
| Polydatin | 25 g |
| Gastrodin | 80 g |
| Sulfobutyl ether-β-cyclodextrin | 30 g |
| Water for injection | 1000 ml |

60% of the total amount of water for injection for preparation was added into a preparation tank, heated to 85 °C with slight boiling, and kept at 80 °C. The vacuum was initiated while stirring for 45 minutes, followed by bringing polydatin along with gastrodin and sulfobutyl ether-β-cyclodextrin weighed according to the amounts in the formula by nitrogen gas into the preparation tank. Polydatin dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 42: Preparation of a powder for injection by dissolving a stilbene compound

**Formula:**

| | |
|---|---|
| Rhapontin | 50 g |
| Gastrodin | 100 g |
| Hydroxypropyl-β-cyclodextrin | 30 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing rhapontin along with gastrodin and hydroxypropyl-β-cyclodextrin weighed according to the amounts in the formula by nitrogen gas into the preparation tank. Rhapontin dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 43: Preparation of a powder for injection by dissolving a stilbene compound

**Formula:**

| | |
|---|---|
| Deoxyrhapontin (methyl polydatin) | 50 g |
| Gastrodin | 100 g |
| Methyl-β-cyclodextrin | 30 g |
| Water for injection | 1000 ml |

60% of the total amount of water for injection for preparation was added into a preparation tank, heated to 98 °C with slight boiling, and kept at 80 °C. The vacuum was initiated while stirring for 15 minutes, followed by bringing deoxyrhapontin (methyl polydatin) along with gastrodin and methyl-β-cyclodextrin weighed according to the amounts in the formula by nitrogen gas into the preparation tank. Deoxyrhapontin (methyl polydatin) dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 44: Preparation of a powder for injection by dissolving a stilbene compound

**Formula:**

| | |
|---|---|
| Resveratrol | 50 g |
| Gastrodin | 100 g |
| Thioether-β-cyclodextrin | 30 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing resveratrol along with gastrodin and thioether-β-cyclodextrin weighed according to the amounts in the formula by nitrogen gas into the preparation tank. Resveratrol dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 45: Preparation of a powder for injection by dissolving a stilbene compound

**Formula:**

| | |
|---|---|
| Piceatannol | 50 g |
| Gastrodin | 100 g |
| Sulfobutyl ether-β-cyclodextrin | 30 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing piceatannol along with gastrodin and sulfobutyl ether-β-cyclodextrin weighed according to the amounts in the formula by nitrogen gas into the preparation tank. Piceatannol dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 46: Preparation of a powder for injection by dissolving a stilbene compound

**Formula:**

| | |
|---|---|
| Polydatin | 5 g |
| Hydroxypropyl-β-cyclodextrin | 30 g |
| Water for injection | 1000 ml |

60% of the total amount of water for injection for preparation was added into a preparation tank, heated to 83 °C with slight boiling, and kept at 80 °C. The vacuum was initiated while stirring for 25 minutes, followed by bringing polydatin along with hydroxypropyl-β-cyclodextrin weighed according to the amounts in the formula by nitrogen gas into the preparation tank. Polydatin dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 47: Preparation of a powder for injection by dissolving a stilbene compound

**Formula:**

| | |
|---|---|
| Triptophenolide | 5 g |
| Methyl-β-cyclodextrin | 30 g |
| Water for injection | 1000 ml |

60% of the total amount of water for injection for preparation was added into a preparation tank, heated to 88 °C with slight boiling, and kept at 80 °C. The vacuum was initiated while stirring for 40 minutes, followed by bringing triptophenolide along with methyl-β-cyclodextrin weighed according to the amounts in the formula by nitrogen gas into the preparation tank. Triptophenolide dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 48: Preparation of a powder for injection by dissolving a stilbene compound

**Formula:**

| | |
|---|---|
| Rhapontin | 5 g |
| Thioether-β-cyclodextrin | 30 g |
| Water for injection | 1000 ml |

60% of the total amount of water for injection for preparation was added into a preparation tank, heated to 92 °C with slight boiling, and kept at 80 °C. The vacuum was initiated while stirring for 40 minutes, followed by bringing rhapontin along with thioether-β-cyclodextrin weighed according to the amounts in the formula by nitrogen gas into the preparation tank. Rhapontin dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 49: Preparation of a powder for injection by dissolving a stilbene compound

**Formula:**

| | |
|---|---|
| Deoxyrhapontin (methyl polydatin) | 5 g |
| Sulfobutyl ether-β-cyclodextrin | 30 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing deoxyrhapontin (methyl polydatin) along with sulfobutyl ether-β-cyclodextrin weighed according to the amounts in the formula by nitrogen gas into the preparation tank. Deoxyrhapontin (methyl polydatin) dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 50: Preparation of a powder for injection by dissolving a stilbene compound

**Formula:**

| | |
|---|---|
| Resveratrol | 10 g |
| Hydroxypropyl-β-cyclodextrin | 30 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing resveratrol along with hydroxypropyl-β-cyclodextrin weighed according to the amounts in the formula by nitrogen gas into the preparation tank. Resveratrol dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 51: Preparation of a powder for injection by dissolving a stilbene compound

**Formula:**

| | |
|---|---|
| Triptophenolide | 10 g |
| Methyl-β-cyclodextrin | 30 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with methyl-β-cyclodextrin weighed according to the amounts in the formula by nitrogen gas into the preparation tank. Triptophenolide dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 52: Preparation of a powder for injection by dissolving a stilbene compound

**Formula:**

| | |
|---|---|
| Triptophenolide | 10 g |
| Thioether-β-cyclodextrin | 30 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with thioether-β-cyclodextrin weighed according to the amounts in the formula by nitrogen gas into the preparation tank. Triptophenolide dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 53: Preparation of a powder for injection by dissolving a stilbene compound

**Formula:**

| | |
|---|---|
| Piceatannol | 10 g |
| Sulfobutyl ether-β-cyclodextrin | 30 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing piceatannol along with sulfobutyl ether-β-cyclodextrin weighed according to the amounts in the formula by nitrogen gas into the preparation tank. Piceatannol dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 54: Preparation of a powder for injection by dissolving a stilbene compound

**Formula:**

| | |
|---|---|
| Polydatin | 10 g |
| Hydroxypropyl-β-cyclodextrin | 50 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing polydatin along with hydroxypropyl-β-cyclodextrin weighed according to the amounts in the formula by nitrogen gas into the preparation tank. Polydatin dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 55: Preparation of a powder for injection by dissolving a stilbene compound

**Formula:**

| | |
|---|---|
| Triptophenolide | 10 g |
| Methyl-β-cyclodextrin | 50 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with methyl-β-cyclodextrin weighed according to the amounts in the formula by nitrogen gas into the preparation tank. Triptophenolide dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 56: Preparation of a powder for injection by dissolving a stilbene compound

**Formula:**

| | |
|---|---|
| Rhapontin | 10 g |
| Thioether-β-cyclodextrin | 50 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing rhapontin along with thioether-β-cyclodextrin weighed according to the amounts in the formula by nitrogen gas into the preparation tank. Rhapontin dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 57: Preparation of a powder for injection by dissolving a stilbene compound

**Formula:**

| | |
|---|---|
| Deoxyrhapontin (methyl polydatin) | 10 g |
| Hydroxypropyl-β-cyclodextrin | 50 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing deoxyrhapontin (methyl polydatin) along with sulfobutyl ether-β-cyclodextrin weighed according to the amounts in the formula by nitrogen gas into the preparation tank. Deoxyrhapontin (methyl polydatin) dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 58: Preparation of a powder for injection by dissolving a stilbene compound

**Formula:**

| | |
|---|---|
| Triptophenolide | 20 g |
| Hydroxypropyl-β-cyclodextrin | 50 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with hydroxypropyl-β-cyclodextrin weighed according to the amounts in the formula by nitrogen gas into the preparation tank. Triptophenolide dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 59: Preparation of a powder for injection by dissolving a stilbene compound

**Formula:**

| | |
|---|---|
| Triptophenolide | 20 g |
| Methyl-β-cyclodextrin | 50 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with methyl-β-cyclodextrin weighed according to the amounts in the formula by nitrogen gas into the preparation tank. Triptophenolide dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 60: Preparation of a powder for injection by dissolving a stilbene compound

**Formula:**

| | |
|---|---|
| Resveratrol | 20 g |
| Thioether-β-cyclodextrin | 50 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing resveratrol along with thioether-β-cyclodextrin weighed according to the amounts in the formula by nitrogen gas into the preparation tank. Resveratrol dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 61: Preparation of a powder for injection by dissolving a stilbene compound

**Formula:**

| | |
|---|---|
| Triptophenolide | 20 g |
| Sulfobutyl ether-β-cyclodextrin | 50 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with sulfobutyl ether-β-cyclodextrin weighed according to the amounts in the formula by nitrogen gas into the preparation tank. Triptophenolide dissolved in about 5 minutes, and then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 62: Preparation of a powder for injection by dissolving a stilbene compound

**Formula:**

| | |
|---|---|
| Piceatannol | 5 g |
| Gastrodin | 100 g |
| Sodium bisulfite | 0.2 g |
| Disodium edetate | 0.1g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing piceatannol along with gastrodin weighed according to the amounts in the formula by nitrogen gas into the preparation tank. Piceatannol dissolved in about 5 minutes, then vacuum and nitrogen gas were removed, and sodium bisulfite and disodium edetate were added, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 63: Preparation of a powder for injection by dissolving a stilbene compound

**Formula:**

| | |
|---|---|
| Triptophenolide | 5 g |
| Gastrodin | 100 g |
| Sodium sulfite | 0.2 g |
| Disodium edetate | 0.1g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, and sodium sulfite and disodium edetate were added, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 64: Preparation of a powder for injection by dissolving a stilbene compound

**Formula:**

| | |
|---|---|
| Polydatin | 5 g |
| Gastrodin | 100 g |
| Vitamin C | 0.2 g |
| Disodium edetate | 0.1g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing polydatin along with gastrodin weighed according to the amounts in the formula by nitrogen gas into the preparation tank. Polydatin dissolved in about 5 minutes, then vacuum and nitrogen gas were removed, and vitamin C and disodium edetate were added, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 65: Preparation of a powder for injection by dissolving a stilbene compound

**Formula:**

| | |
|---|---|
| Triptophenolide | 5 g |
| Gastrodin | 100 g |
| L-cysteine hydrochloride | 0.2 g |
| Disodium edetate | 0.1g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, and L-cysteine hydrochloride and disodium edetate were added, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 66: Preparation of a powder for injection by dissolving a stilbene compound

**Formula:**

| | |
|---|---|
| Rhapontin | 5 g |
| Gastrodin | 100 g |
| Glutathione | 0.2 g |
| Disodium edetate | 0.1g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing rhapontin along with gastrodin weighed according to the amounts in the formula by nitrogen gas into the preparation tank. Rhapontin dissolved in about 5 minutes, then vacuum and nitrogen gas were removed, and glutathione and disodium edetate were added, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 67: Preparation of a powder for injection by dissolving a stilbene compound

**Formula:**

| | |
|---|---|
| Deoxyrhapontin (methyl polydatin) | 5 g |
| Gastrodin | 100 g |
| Glutathione sodium | 0.2 g |
| Disodium edetate | 0.1g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing deoxyrhapontin (methyl polydatin) along with gastrodin weighed according to the amounts in the formula by nitrogen gas into the preparation tank. Deoxyrhapontin (methyl polydatin) dissolved in about 5 minutes, then vacuum and nitrogen gas were removed, and glutathione sodium and disodium edetate were added, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 68: Preparation of a powder for injection by dissolving a stilbene compound

**Formula:**

| | |
|---|---|
| Triptophenolide | 50 g |
| Hydroxypropyl-β-cyclodextrin | 80 g |
| Sodium bisulfite | 0.2 g |
| Disodium edetate | 0.1g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with hydroxypropyl-β-cyclodextrin weighed according to the amounts in the formula by nitrogen gas into the preparation tank. Triptophenolide dissolved in about 5 minutes, then vacuum and nitrogen gas were removed, and sodium bisulfite and disodium edetate were added, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 69: Preparation of a powder for injection by dissolving a stilbene compound

**Formula:**

| | |
|---|---|
| Triptophenolide | 50 g |
| Methyl-β-cyclodextrin | 80 g |
| Sodium sulfite | 0.2 g |
| Disodium edetate | 0.1g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with methyl-β-cyclodextrin weighed according to the amounts in the formula by nitrogen gas into the preparation tank. Triptophenolide dissolved in about 5 minutes, then vacuum and nitrogen gas were removed, and sodium sulfite and disodium edetate were added, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 70: Preparation of a powder for injection by dissolving a stilbene compound

**Formula:**

| | |
|---|---|
| Triptophenolide | 50 g |
| Thioether-β-cyclodextrin | 80 g |
| Vitamin C | 0.2 g |
| Disodium edetate | 0.1g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with thioether-β-cyclodextrin weighed according to the amounts in the formula by nitrogen gas into the preparation tank. Triptophenolide dissolved in about 5 minutes, then vacuum and nitrogen gas were removed, and vitamin C and disodium edetate were added, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 71: Preparation of a powder for injection by dissolving a stilbene compound

**Formula:**

| | |
|---|---|
| Triptophenolide | 50 g |
| Sulfobutyl ether-β-cyclodextrin | 80 g |
| L-cysteine hydrochloride | 0.2 g |
| Disodium edetate | 0.1 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with hydroxypropyl-β-cyclodextrin weighed according to the amounts in the formula by nitrogen gas into the preparation tank. Triptophenolide dissolved in about 5 minutes, then vacuum and nitrogen gas were removed, and L-cysteine hydrochloride and disodium edetate were added, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 72: Preparation of a powder for injection by dissolving a stilbene compound

**Formula:**

| | |
|---|---|
| Triptophenolide | 50 g |
| Hydroxypropyl-β-cyclodextrin | 80 g |
| Glutathione | 0.2 g |
| Disodium edetate | 0.1g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with hydroxypropyl-β-cyclodextrin weighed according to the amounts in the formula by nitrogen gas into the preparation tank. Triptophenolide dissolved in about 5 minutes, then vacuum and nitrogen gas were removed, and glutathione and disodium edetate were added, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 73: Preparation of a powder for injection by dissolving a stilbene compound

**Formula:**

| | |
|---|---|
| Triptophenolide | 50 g |
| Hydroxypropyl-β-cyclodextrin | 80 g |
| Glutathione sodium | 0.2 g |
| Disodium edetate | 0.1g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with hydroxypropyl-β-cyclodextrin weighed according to the amounts in the formula by nitrogen gas into the preparation tank. Triptophenolide dissolved in about 5 minutes, then vacuum and nitrogen gas were removed, and glutathione sodium and disodium edetate were added, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder for injection.

### Example 74: Preparation of a powder for injection by dissolving a stilbene compound

**Formula:**

| | |
|---|---|
| Triptophenolide | 5 g |
| Gastrodin | 0.5 g |
| Water for injection | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with gastrodin weighed according to the amounts in the formula by nitrogen gas into the preparation tank. Triptophenolide dissolved in about 5 minutes, then vacuum and nitrogen gas were removed, followed by adjusting pH and content by adding sodium hydroxide or hydrochloric acid, and adding water to the total amount, thus completing the preparation process. Next, it was sterile-filtered, sterile-filled and freeze-dried by a conventional process to obtain the freeze-dried powder.

### Example 75: Solubility of triptophenolide in aqueous solutions with different concentrations of gastrodin and/or water-soluble cyclodextrin

Results of solubility were shown in Table 1.

**Table 1. Solubility of triptophenolide in aqueous solutions with different concentrations of gastrodin and/or water-soluble cyclodextrin**

| Gastrodin concentration (%) | Triptophenolide solubility (mg/ml) | Hydroxypropyl-β-cyclodextrin concentration (%) | Triptophenolide solubility (mg/ml) | Solubilizer concentration (%) | | Triptophenolide solubility (mg/ml) |
|---|---|---|---|---|---|---|
| | | | | Gastrodin | Hydroxypropyl-β-cyclodextrin | |
| 0 | 1.752 | 0 | 1.752 | 0 | 3 | 10.785 |
| 1 | 3.191 | 1 | 4.796 | 1 | 3 | 14.786 |
| 3 | 5.097 | 3 | 10.785 | 3 | 3 | 16.215 |
| 5 | 7.107 | 5 | 20.028 | 5 | 3 | 22.61 |
| 8 | 9.169 | 8 | 26.142 | 8 | 3 | 30.945 |
| 10 | 11.438 | 10 | 34.093 | 10 | 3 | 39.811 |
| 15 | 11.761 | 15 | 43.264 | 15 | 3 | 55.987 |
| 25 | 38.378 | 20 | 59.198 | 20 | 3 | 107.193 |
| 30 | 60.424 | 25 | 64.329 | 25 | 3 | 126.647 |
| 35 | 87.793 | 30 | 116.235 | 30 | 3 | 181.272 |
| 40 | 85.117 | 35 | 159.444 | 35 | 3 | 233.379 |
| 45 | 97.993 | 40 | 188.611 | 40 | 3 | 197.257 |
| 50 | 105.017 | 45 | 180.509 | 45 | 3 | 186.789 |
| - | - | 50 | 183.10 | 50 | 3 | 188.029 |

It can be seen from Table 1 that gastrodin and/or water-soluble cyclodextrin can improve the solubility of triptophenolide in water.

### Example 76: Redissolution tests on freeze-dried powders prepared by dissolving flavonoid compounds (comparative example)

2 ml of water for injection was injected into the freeze-dried powder prepared in Example 2 to Example 9 respectively, and redissolution was investigated over time without shaking. Test results were shown in Table 2.

**Table 2. Results of redissolution tests on freeze-dried powders prepared by dissolving flavonoid compounds**

| Groups | | Dissolusion time (s) |
|---|---|---|
| Test group | Example 2 | 4 |
| | Example 3 | 4 |
| | Example 4 | 5 |
| | Example 5 | 5 |
| | Example 6 | 4 |
| | Example 7 | 5 |
| | Example 8 | 4 |
| | Example 9 | 4 |
| Control group | A sample prepared in a mixed solvent of PEG400 and mannitol | 32 |

The redissolution tests showed that the freeze-dried powder of flavonoid compound prepared by freeze-drying the water-soluble drug using gastrodin as the solubilizer dissolved completely in only 5 seconds, and had a significant increase in the redissolution speed as compared with the control group, which meets the requirement for clinical emergencies.

### Example 77: Redissolution tests on freeze-dried powders prepared by dissolving carbon glycoside compounds

2 ml of water for injection was injected into the freeze-dried powder prepared in Example 17 to Example 25, Example 32, Example 33 and Example 34 respectively, and redissolution was investigated over time without shaking. Test results were shown in Table 3.

**Table 3. Results of Redissolution tests on freeze-dried powders prepared by dissolving carbon glycoside compounds**

| Groups | | Dissolution time (s) |
|---|---|---|
| Test group | Example 17 | 2 |
| | Example 18 | 2 |
| | Example 19 | 2 |
| | Example 20 | 2 |
| | Example 21 | 2 |
| | Example 22 | 2 |
| | Example 23 | 2 |
| | Example 24 | 2 |
| | Example 25 | 2 |
| | Example 32 | 2 |
| | Example 33 | 2 |
| | Example 34 | 2 |
| Control group | A sample prepared in a mixed solvent of PEG400 and mannitol | 30 |

The redissolution tests showed that the freeze-dried powder of the carbon glycoside compound prepared by freeze-drying the water-soluble drug using gastrodin as the solubilizer dissolved completely in only 2 seconds, and had a significant increase in the redissolution speed as compared with the control group, which meets the requirement for clinical emergencies.

### Example 78: Redissolution tests on freeze-dried powders prepared by dissolving stilbene compounds

2 ml of water for injection was injected into the freeze-dried powder prepared in Example 35 to Example 74 respectively, and redissolution was investigated over time without shaking. Test results were shown in Table 4.

**Table 4. Results of Redissolution tests on freeze-dried powders prepared by dissolving stilbene compounds**

| Groups | | Dissolusion time (s) |
|---|---|---|
| Test group | Example 35 | 3 |
| | Example 36 | 2 |
| | Example 37 | 2 |
| | Example 38 | 2 |
| | Example 39 | 2 |
| | Example 40 | 3 |
| | Example 41 | 2 |
| | Example 42 | 2 |
| | Example 43 | 3 |
| | Example 44 | 3 |
| | Example 45 | 2 |
| | Example 46 | 2 |
| | Example 47 | 3 |
| | Example 48 | 3 |
| | Example 49 | 3 |
| | Example 50 | 2 |
| | Example 51 | 2 |
| | Example 52 | 3 |
| | Example 53 | 2 |
| | Example 54 | 2 |
| | Example 55 | 3 |
| | Example 56 | 2 |
| | Example 57 | 3 |
| | Example 58 | 2 |
| | Example 59 | 2 |
| | Example 60 | 3 |
| | Example 61 | 3 |
| | Example 62 | 2 |
| | Example 63 | 2 |
| | Example 64 | 2 |
| | Example 65 | 2 |
| | Example 66 | 2 |
| | Example 67 | 3 |
| | Example 68 | 3 |
| | Example 69 | 2 |
| | Example 70 | 3 |
| | Example 71 | 2 |
| | Example 72 | 3 |
| | Example 73 | 3 |
| | Example 74 | 3 |
| Control group | A sample prepared in a mixed solvent of PEG400 and mannitol | 26 |

The redissolution tests showed that the freeze-dried powder of the stilbene compound prepared by freeze-drying the water-soluble drug using gastrodin as the solubilizer dissolved completely in only 3 seconds, and had a significant increase in the redissolution speed as compared with the control group, which meets the requirement for clinical emergencies.

### Example 79: Stability tests

Stability tests were performed on the freeze-dried powders prepared according to the present invention or as disclosed herein.

High-temperature test: the injections of the flavonoid compounds prepared in Example 2 to Example 16, the injections of the carbon glycoside compounds prepared in Example 17 to Example 34, and the injections of the stilbene compounds prepared in Example 35 to Example 74 were placed into an incubator of 60 °C respectively, and taken out and detected according to the investigation items on day 5 and 10. Test results were shown in Table 5 to Table 7.

**Table 5. High-temperature test results of injections of the flavonoid compounds (comparative data)**

| Groups | | Conditions | Properties | Related substances (total area of related substances) | pH | Content (%) |
|---|---|---|---|---|---|---|
| Control group | A sample prepared in a mixed solvent of PEG400 and mannitol | Before placed | Light yellow | Less than the main peak area of control solution | 6.21 | 99.8 |
| | | 60 °C, 5 days | Light yellow | Less than the main peak area of control solution | 5.21 | 94.48 |
| | | 60 °C, 10 days | Light yellow | Less than the main peak area of control solution | 4.72 | 88.65 |
| Test group | Freeze-dried powder of water-soluble flavonoid compounds | Before placed | Light yellow | Less than the main peak area of control solution | 6.60 | 100.65-101.01 |
| | | 60 °C, 5 days | Light yellow | Less than the main peak area of control solution | 6.44 | 99.97-100.01 |
| | | 60 °C, 10 days | Light yellow | Less than the main peak area of control solution | 6.30 | 98.65-98.68 |

**Table 6. High-temperature test results of injections of the carbon glycoside compounds**

| Groups | | Conditions | Properties | Related substances (total area of related substances) | pH | Content (%) |
|---|---|---|---|---|---|---|
| Control group | A sample prepared in a mixed solvent of PEG400 and mannitol | Before placed | Light yellow | Less than the main peak area of control solution | 6.05 | 99.5 |
| | | 60 °C, 5 days | Light yellow | Less than the main peak area of control solution | 5.05 | 93.01 |
| | | 60 °C, 10 days | Light yellow | Less than the main peak area of control solution | 4.08 | 86.72 |
| Test group | Freeze-dried powder of water-soluble carbon glycoside compounds | Before placed | Light yellow | Less than the main peak area of control solution | 6.55 | 100.02-100.07 |
| | | 60 °C, 5 days | Light yellow | Less than the main peak area of control solution | 6.32 | 99.68-99.78 |
| | | 60 °C, 10 days | Light yellow | Less than the main peak area of control solution | 6.24 | 97.96-98.75 |

**Table 7. High-temperature test results of injections of the stilbene compounds**

| Groups | | Conditions | Properties | Related substances (total area of related substances) | pH | Content (%) |
|---|---|---|---|---|---|---|
| Control | A sample prepared in a | Before placed | Off-white | Less than the main peak area of control solution | 4.07 | 94.13 |
| | | 60 °C, 5 days | Light red | Less than the main peak area of control solution | 4.15 | 83.01 |
| | | 60 °C, 10 days | Light red | Less than the main peak area of control solution | 3.98 | 74.51 |
| Test group | Freeze-dried powder of water-soluble stilbene compounds | Before placed | White | Less than the main peak area of control solution | 5.41 | 100.77-100.96 |
| | | 60 °C, 5 days | White | Less than the main peak area of control solution | 5.09 | 98.75-99.87 |
| | | 60 °C, 10 days | White | Less than the main peak area of control solution | 5.01 | 95.88-98.27 |

Strong light exposure tests: the injections of the flavonoid compounds prepared in Example 2 to Example 16, the injections of the carbon glycoside compounds prepared in Example 17 to Example 34, and the injections of the stilbene compounds prepared in Example 35 to Example 74 were placed into a light cabinet with a illuminance of 4600 LX, and taken out and detected according to the investigation items on day 5 and 10. Test results were shown in table 8 to table 10.

**Table 8. Strong light exposure test results of injections of the flavonoid compounds**

| **(comparative data)** | | | | | | |
|---|---|---|---|---|---|---|
| Groups | | Conditions | Properties | Related substances (total area of related substances) | pH | Content (%) |
| Control group | A sample prepared in a mixed solvent of PEG400 and mannitol | Before placed | Light yellow | Less than the main peak area of control solution | 6.21 | 99.8 |
| | | Light exposure for 5 days | Light yellow | Less than the main peak area of control solution | 6.05 | 98.15 |
| | | Light exposure for 10 days | Light yellow | Less than the main peak area of control solution | 5.28 | 89.85 |
| Test group | Freeze-dried powder of water-soluble flavonoid compounds | Before placed | Light yellow | Less than the main peak area of control solution | 6.60 | 100.65-101.01 |
| | | Light exposure for 5 days | Light yellow | Less than the main peak area of control solution | 6.54 | 99.68-99.92 |
| | | Light exposure for 10 days | Light yellow | Less than the main peak area of control solution | 6.40 | 98.04-99.01 |

**Table 9. Strong light exposure test results of injections of the carbon glycoside compounds**

| Groups | | Conditions | Properties | Related substances (total area of related substances) | pH | Content (%) |
|---|---|---|---|---|---|---|
| Control group | A sample prepared in a mixed solvent of PEG400 and mannitol | Before placed | Light yellow | Less than the main peak area of control solution | 6.05 | 99.5 |
| | | Light exposure for 5 days | Light yellow | Less than the main peak area of control solution | 5.05 | 95.28 |
| | | Light exposure for 10 days | Light yellow | Less than the main peak area of control solution | 4.82 | 90.55 |
| Test group | Freeze-dried powder of water-soluble carbon glycoside compounds | Before placed | Light yellow | Less than the main peak area of control solution | 6.55 | 100.02-100.07 |
| | | Light exposure for 5 days | Light yellow | Less than the main peak area of control solution | 6.40 | 99.05-99.86 |
| | | Light exposure for 10 days | Light yellow | Less than the main peak area of control solution | 6.32 | 98.88-98.96 |

**Table 10. Strong light exposure test results of injections of the stilbene compounds**

| Groups | | Conditions | Properties | Related substances (total area of related substances) | pH | Content (%) |
|---|---|---|---|---|---|---|
| Control group | A sample prepared in a mixed solvent of PEG400 and mannitol | Before placed | Off-white | Less than the main peak area of control solution | 4.07 | 94.13 |
| | | Light exposure for 5 days | Light yellow | Less than the main peak area of control solution | 4.09 | 80.88 |
| | | Light exposure for 10 days | Light yellow | Less than the main peak area of control solution | 3.82 | 77.55 |
| Test group | Freeze-dried powder of water-soluble stilbene compounds | Before placed | Light yellow | Less than the main peak area of control solution | 6.60 | 100.77-100.96 |
| | | Light exposure for 5 days | Light yellow | Less than the main peak area of control solution | 6.54 | 95.68-96.01 |
| | | Light exposure for 10 days | Light yellow | Less than the main peak area of control solution | 6.40 | 92.69-94.79 |

Stability tests showed that the degree of content degradation for the traditional sample prepared in a mixed solvent of PEG400 and mannitol was above 8%, failing to meet the requirements of stability for pharmaceutical preparations; while in the present invention, the freeze-dried powder prepared by using gastrodin as the solubilizer for the flavonoid compound, the carbon glycoside compound or the stilbene compound, compared with that before placed, has no change in properties and related substances, and has no significant change in pH and content.

### Example 80: Stability tests

**Table 11. Stability tests**

| Groups | 1 | 2 | 3 | 4 | 5 |
|---|---|---|---|---|---|
| Triptophenolide | 10 g | 10 g | 10 g | 10 g | 10 g |
| Hydroxypropyl-β-cyclodextrin | 30 g | 30 g | 30 g | 30 g | 30 g |
| Anti-oxidant | Vitamin C 0.2 g | L-cysteine hydrochloride 0.2 g | Glutathione sodium 0.2 g | Sodium bisulfite 0.2 g | EDTA2Na 0.2 g |
| Sodium hydroxide or hydrochloric acid | Suitable amount | Suitable amount | Suitable amount | Suitable amount | Suitable amount |
| Water for injection | 1000 ml | 1000 ml | 1000 ml | 1000 ml | 1000 ml |

60% of the total amount of water for injection for preparation was added into a preparation tank, heated to 95 °C with slight boiling, and kept at 80 °C. The vacuum was initiated while stirring for 10 minutes, followed by bringing triptophenolide along with hydroxypropyl-β-cyclodextrin weighed according to the amounts in the formula by nitrogen gas into the preparation tank. Triptophenolide dissolved in about 5 minutes, then vacuum and nitrogen gas were removed, and vitamin C, L-cysteine hydrochloride, glutathione sodium, sodium bisulfite and EDTA2Na were respectively added into 5 preparation tanks, followed by adjusting pH and content and adding water to the total amount, thus completing the preparation process. Next, they were sterile-filtered and filled by a conventional process to obtain the injections, which were used as experimental solutions. A solution free of anti-oxidants was prepared as the control solution by the same method. 10% hydrogen peroxide solution was added respectively, and heated at 85 °C for 60 h. The injections and control solution both had relatively good stability. When taken out, it was observed that all the injections containing the anti-oxidant were colorless and transparent liquid, while the injections free of the anti-oxidant had a slightly yellow color, so the addition of an anti-oxidant can improve the stability of the injection.

## Claims

1. A method for dissolving a carbon glycoside compound or a stilbene compound, **characterized in that** the method comprises the following steps:
heating water for injection to 80-98 °C, followed by stirring under vacuum condition at 30-80 °C, and then adding the carbon glycoside compound or the stilbene compound along with a solubilizer under the protection of nitrogen gas or argon gas to obtain a solution of said compounds;
wherein the solubilizer is one or a mixture of two or more of gastrodin, water-soluble cyclodextrin and ethanol, and wherein the water-soluble cyclodextrin is one or a mixture of two or more of methyl-β-cyclodextrin, thioether-β-cyclodextrin and sulfobutyl ether-β-cyclodextrin; and
wherein the mass ratio of the carbon glycoside compound or the stilbene compound to the solubilizer is 1:1-20.

2. The method according to claim 1, **characterized in that** the time for stirring is 1-60 min.

3. The method according to claim 1, **characterized in that** the mass ratio of the carbon glycoside compound or the stilbene compound to the solubilizer is 1 :3-10

4. The method according to claim 1, **characterized in that** the mass-to-volume ratio of the solubilizer to the solution of the reactant is 2%-60% in g/mL.

5. The method according to claim 1, **characterized in that** the mass-to-volume ratio of the solubilizer to the solution of the reactant is 3%-50% in g/mL.

6. The method according to claim 1, **characterized in that** the mass-to-volume ratio of the solubilizer to the solution of the reactant is 3%-28% in g/mL.

7. A method for preparing an injection of a carbon glycoside compound or a stilbene compound, **characterized in that** the method comprises the following steps:
heating water for injection to 80-98 °C, followed by stirring under vacuum condition at 30-80 °C, and then adding the carbon glycoside compound or the stilbene compound along with a solubilizer under the protection of nitrogen gas or argon gas to obtain a solution of said compounds; and adjusting pH value, filtering, filling and sterilizing to obtain the injection;
wherein the solubilizer is one or a mixture of two or more of gastrodin, water-soluble cyclodextrin and ethanol, and wherein the water-soluble cyclodextrin is one or a mixture of two or more of methyl-β-cyclodextrin, thioether-β-cyclodextrin and sulfobutyl ether-β-cyclodextrin; and
wherein the mass ratio of the carbon glycoside compound or the stilbene compound to the solubilizer is 1:1-20.

8. A method for preparing a powder for injection of a carbon glycoside compound or a stilbene compound, **characterized in that** the method comprises the following steps:
heating water for injection to 80-98 °C, followed by stirring under vacuum conditions at 30-80 °C, and then adding the carbon glycoside compound or the stilbene compound along with a solubilizer under the protection of nitrogen gas or argon gas to obtain a solution of said compounds; and adjusting pH value, sterile-filtering, sterile-filling and freeze-drying to obtain the powder for injection,
wherein the solubilizer is one or a mixture of two or more of gastrodin, water-soluble cyclodextrin and ethanol, and wherein the water-soluble cyclodextrin is one or a mixture of two or more of methyl-β-cyclodextrin, thioether-β-cyclodextrin and sulfobutyl ether-β-cyclodextrin; and
wherein the mass ratio of the carbon glycoside compound or the stilbene compound to the solubilizer is 1:1-20.

## Patentansprüche

1. Verfahren zum Lösen einer Kohlenstoffglykosidverbindung oder einer Stilbenverbindung, **dadurch gekennzeichnet, dass** das Verfahren die folgenden Schritte umfasst:
Erwärmen von Wasser zur Injektion auf 80-98 °C, gefolgt von Rühren unter Vakuumbedingung bei 30-80 °C, und dann Zugeben der Kohlenstoffglykosidverbindung oder der Stilbenverbindung zusammen mit einem Lösungsvermittler unter dem Schutz von Stickstoffgas oder Argongas, um eine Lösung dieser Verbindungen zu erhalten;
wobei der Lösungsvermittler eines oder eine Mischung aus zwei oder mehreren von Gastrodin, wasserlöslichem Cyclodextrin und Ethanol ist und wobei das wasserlösliche Cyclodextrin eines oder eine Mischung aus zwei oder mehreren von Methyl-β-cyclodextrin, Thioether-β-cyclodextrin und Sulfobutylether-β-cyclodextrin ist; und
wobei das Masseverhältnis der Kohlenstoffglykosidverbindung oder der Stilbenverbindung zu dem Lösungsvermittler 1:1-20 ist.

2. Verfahren gemäß Anspruch 1, **dadurch gekennzeichnet, dass** die Zeit zum Rühren 1-60 min ist.

3. Verfahren gemäß Anspruch 1, **dadurch gekennzeichnet, dass** das Masseverhältnis der Kohlenstoffglykosidverbindung oder der Stilbenverbindung zu dem Lösungsvermittler 1:3-10 ist.

4. Verfahren gemäß Anspruch 1, **dadurch gekennzeichnet, dass** das Masse-zu-Volumen-Verhältnis des Lösungsvermittlers zu der Lösung des Reaktanten 2 % - 60 % in g/mL ist.

5. Verfahren gemäß Anspruch 1, **dadurch gekennzeichnet, dass** das Masse-zu-Volumen-Verhältnis des Lösungsvermittlers zu der Lösung des Reaktanten 3 % - 50 % in g/mL ist.

6. Verfahren gemäß Anspruch 1, **dadurch gekennzeichnet, dass** das Masse-zu-Volumen-Verhältnis des Lösungsvermittlers zu der Lösung des Reaktanten 3 % - 28 % in g/mL ist.

7. Verfahren zum Herstellen einer Injektion einer Kohlenstoffglykosidverbindung oder einer Stilbenverbindung, **dadurch gekennzeichnet, dass** das Verfahren die folgenden Schritte umfasst:
Erwärmen von Wasser zur Injektion auf 80-98 °C, gefolgt von Rühren unter Vakuumbedingung bei 30-80 °C, und dann Zugeben der Kohlenstoffglykosidverbindung oder der Stilbenverbindung zusammen mit einem Lösungsvermittler unter dem Schutz von Stickstoffgas oder Argongas, um eine Lösung der Verbindungen zu erhalten; und Einstellen des pH-Wertes, Filtrieren, Abfüllen und Sterilisieren, um die Injektion zu erhalten;
wobei der Lösungsvermittler eines oder eine Mischung aus zwei oder mehreren von Gastrodin, wasserlöslichem Cyclodextrin und Ethanol ist und wobei das wasserlösliche Cyclodextrin eines oder eine Mischung aus zwei oder mehreren von Methyl-β-cyclodextrin, Thioether-β-cyclodextrin und Sulfobutylether-β-cyclodextrin ist; und
wobei das Masseverhältnis der Kohlenstoffglykosidverbindung oder der Stilbenverbindung zu dem Lösungsvermittler 1:1-20 ist.

8. Verfahren zum Herstellen eines Pulvers zur Injektion einer Kohlenstoffglykosidverbindung oder einer Stilbenverbindung, **dadurch gekennzeichnet, dass** das Verfahren die folgenden Schritte umfasst:
Erwärmen von Wasser zur Injektion auf 80-98 °C, gefolgt von Rühren unter Vakuumbedingungen bei 30-80 °C, und dann Zugeben der Kohlenstoffglykosidverbindung oder der Stilbenverbindung zusammen mit einem Lösungsvermittler unter dem Schutz von Stickstoffgas oder Argongas, um eine Lösung der Verbindungen zu erhalten; und Einstellen des pH-Wertes, Sterilfiltrieren, Sterilabfüllen und Gefriertrocknen, um das Pulver zur Injektion zu erhalten,
wobei der Lösungsvermittler eines oder eine Mischung aus zwei oder mehreren von Gastrodin, wasserlöslichem Cyclodextrin und Ethanol ist und wobei das wasserlösliche Cyclodextrin eines oder eine Mischung aus zwei oder mehreren von Methyl-β-cyclodextrin, Thioether-β-cyclodextrin und Sulfobutylether-β-cyclodextrin ist; und
wobei das Masseverhältnis der Kohlenstoffglykosidverbindung oder der Stilbenverbindung zu dem Lösungsvermittler 1:1-20 ist.

## Revendications

1. Procédé pour dissoudre un composé glycoside carboné ou un composé stilbène, le procédé étant **caractérisé en ce qu'**il comprend les étapes consistant à :
chauffer à 80-98°C de l'eau pour injection, puis agiter dans des conditions sous vide à 30-80°C, et ensuite ajouter le composé glycoside carboné ou le composé stilbène conjointement avec un solubilisant sous protection d'azote gazeux ou d'argon gazeux pour obtenir une solution desdits composés ;
dans lequel le solubilisant est l'un ou un mélange de deux ou plus de deux parmi la gastrodine, la cyclodextrine soluble dans l'eau et l'éthanol, et dans lequel la cyclodextrine soluble dans l'eau est l'une ou un mélange de deux ou plus de deux parmi la méthyl-β-cyclodextrine, la thioéther-β-cyclodextrine et la sulfobutyléther-β-cyclodextrine ; et
dans lequel le rapport en masse du composé glycoside carboné ou du composé stilbène au solubilisant est de 1:1 à 20.

2. Procédé selon la revendication 1, **caractérisé en ce que** le temps d'agitation est de 1 à 60 minutes.

3. Procédé selon la revendication 1, **caractérisé en ce que** le rapport en masse du composé glycoside carboné ou du composé stilbène au solubilisant est de 1:3 à 10.

4. Procédé selon la revendication 1, **caractérisé en ce que** le rapport masse/volume du solubilisant à la solution du réactif est de 2 % à 60 % en g/ml.

5. Procédé selon la revendication 1, **caractérisé en ce que** le rapport masse/volume du solubilisant à la solution du réactif est de 3 % à 50 % en g/ml.

6. Procédé selon la revendication 1, **caractérisé en ce que** le rapport masse/volume du solubilisant à la solution du réactif est de 3 % à 28 % en g/ml.

7. Procédé pour préparer une composition injectable d'un composé glycoside carboné ou d'un composé stilbène, le procédé étant **caractérisé en ce qu'**il comprend les étapes consistant à :
chauffer à 80-98°C de l'eau pour injection, puis agiter dans des conditions sous vide à 30-80°C, et ensuite ajouter le composé glycoside carboné ou le composé stilbène conjointement avec un solubilisant sous protection d'azote gazeux ou d'argon gazeux pour obtenir une solution desdits composés ; et ajuster la valeur de pH, filtrer, remplir et stériliser pour obtenir la composition injectable ;
dans lequel le solubilisant est l'un ou un mélange de deux ou plus de deux parmi la gastrodine, la cyclodextrine soluble dans l'eau et l'éthanol, et dans lequel la cyclodextrine soluble dans l'eau est l'une ou un mélange de deux ou plus de deux parmi la méthyl-β-cyclodextrine, la thioéther-β-cyclodextrine et la sulfobutyléther-β-cyclodextrine ; et
dans lequel le rapport en masse du composé glycoside carboné ou du composé stilbène au solubilisant est de 1:1 à 20.

8. Procédé pour préparer une poudre pour injection d'un composé glycoside carboné ou d'un composé stilbène, le procédé étant **caractérisé en ce qu'**il comprend les étapes consistant à :
chauffer à 80-98°C de l'eau pour injection, puis agiter dans des conditions sous vide à 30-80°C, et ensuite ajouter le composé glycoside carboné ou le composé stilbène conjointement avec un solubilisant sous protection d'azote gazeux ou d'argon gazeux pour obtenir une solution desdits composés ; et ajuster la valeur de pH, filtrer dans des conditions stériles, remplir dans des conditions stériles et lyophiliser pour obtenir la poudre pour injection ;
dans lequel le solubilisant est l'un ou un mélange de deux ou plus de deux parmi la gastrodine, la cyclodextrine soluble dans l'eau et l'éthanol, et dans lequel la cyclodextrine soluble dans l'eau est l'une ou un mélange de deux ou plus de deux parmi la méthyl-β-cyclodextrine, la thioéther-β-cyclodextrine et la sulfobutyléther-β-cyclodextrine ; et
dans lequel le rapport en masse du composé glycoside carboné ou du composé stilbène au solubilisant est de 1:1 à 20.
